# EUROPÄISCHE PATENTANMELDUNG

(11) **EP 2 108 361 A1**
(43) Veröffentlichungstag der Anmeldung: **14.10.2009**
(21) Anmeldenummer: 09157245.3
(22) Anmeldetag: 03.04.2009
(51) Int. Cl.: A61K 9/107, A61K 9/127, A61K 9/00

(54) **Emulgierende Mikropartikel aus Lipiden zur Stabilisierung lipophiler Wirkstoffe für die kosmetische Anwendung**

(30) Priorität: 09.04.2008 DE 102008018108; 19.09.2008 DE 102008048146
(71) Anmelder: ROVI Cosmetics International GmbH, 36381 Schlüchtern (DE)
(72) Erfinder: Teichmüller, Dirk, 63589 Linsengericht-Geislitz (DE); Blume, Gabriele, 36396 Steinau an der Straße (DE); Sacher, Michael, 36381 Schlüchtern-Breitenbach (DE)
(74) Vertreter: WSL Patentanwälte

(57) **Zusammenfassung**

Um eine Wirkstoffformulierung für kosmetische oder dermatologische Zubereitungen bereitzustellen, mit deren Hilfe lipophile Wirkstoffe so formuliert werden können, daß diese vor der Anwendung vor der Einwirkung von Wasser geschützt sind, wird erfindungsgemäß eine Zusammensetzung mit einer wasserfreien Wirkstoffträgermatrix und einem in dieser Wirkstoffträgermatrix eingeschlossenen Wirkstoff vorgeschlagen, wobei die Zusammensetzung
a) 25 bis 60 Gew.-% Phospholipide, wobei die Phospholipide
i) zu 80 bis 95 Gew.-% gesättigte Phospholipide und
ii) zu 5 bis 20 Gew.-% ungesättigte Phospholipide sind,

b) 20 bis 60 Gew.-% wenigstens ein Wachs,
c) wahlweise 5 bis 10 Gew.-% wenigstens einen Hilfsstoff und
d) 1 bis 20 Gew.-% wenigstens einen lipophilen Wirkstoff

enthält, wobei die Komponenten a) bis c) die Wirkstoffträgermatrix bilden.
Weiterhin betrifft die Anmeldung eine kosmetische oder dermatologische Zubereitung mit einer wasserhaltigen Zubereitungsbasis, vorzugsweise ein Gel, und mit einer darin suspendierten Zusammensetzung der oben genannten Art. Ferner wird ein Verfahren zur Herstellung der erfindungsgemäßen Zusammensetzung und der erfindungsgemäßen Zubereitung vorgestellt.

## Beschreibung

Die vorliegende Erfindung betrifft eine kosmetische oder dermatologische Zusammensetzung mit einer wasserfreien Wirkstoffträgermatrix und wenigstens einem in dieser Wirkstoffträgermatrix eingeschlossenen lipophilen Wirkstoff, wobei die Wirkstoffträgermatrix Anteile an Lipiden aufweist.

Weiterhin betrifft die vorliegende Anmeldung eine kosmetische oder dermatologische Zubereitung, bevorzugt eine Gelformulierung, mit einer wasserhaltigen Zubereitungsbasis und mit in dieser Zubereitungsbasis suspendierten Partikeln, die aus einer Zusammensetzung mit einer wasserfreien Wirkstoffträgermatrix und wenigstens einem in dieser Wirkstoffträgermatrix eingeschlossenen lipophilen Wirkstoff bestehen, wobei die Wirkstoffträgermatrix Anteile an Lipiden aufweist.

Ferner wird ein Verfahren zur Herstellung einer Zusammensetzung mit einer wasserfreien Wirkstoffträgermatrix und wenigstens einem in dieser Wirkstoffträgermatrix eingeschlossenen lipophilen Wirkstoff beschrieben, wobei die Wirkstoffträgermatrix einen Anteil an Lipiden aufweist.

Viele kosmetisch oder dermatologisch relevante Wirkstoffe bzw. Arzneistoffe sind lipophil. Für solche lipophilen Wirkstoffe bzw. Arzneistoffe sind entsprechend geeignete Trägersysteme erforderlich.

Bekannte Wirkstoffformulierungen für kosmetische oder dermatologische Zubereitungen, mit deren Hilfe lipophile Wirkstoffe formuliert werden können, verwenden beispielsweise Liposomen oder Cyclodextrine, in denen die jeweiligen Wirkstoffe verkapselt vorliegen.

Ein weiteres mögliches Trägersystem für lipophile Wirkstoffe sind Lipidpartikel, die beispielsweise eine Größe im Nanometerbereich aufweisen (Nanopartikel, durchschnittlicher Durchmesser = 50 bis 500 nm) können, teilweise aber auch mit einer Größe im Mikrometerbereich (Mikropartikel, durchschnittlicher Durchmesser = 500 bis 2000 µm) verwendet werden.

Die meisten der derzeit eingesetzten Lipidpartikel, die mit einem lipophilen Wirkstoff beladen werden können, sind Nanopartikel, die in hochkonzentrierter Dispersion vorliegen. Als besonders vorteilhaft haben sich bei diesen Nanopartikeln zwei Ausführungsformen erwiesen, von denen die eine aus festen Lipidnanopartikeln (SLN) besteht, die aus einer bei Raumtemperatur festen Lipidkomponente bestehen (EP 0 605 497), während die andere Ausführungsform als nanostrukturierte Lipidcarrier (NLC) bezeichnet wird, die durch eine feste Komponente und eine flüssige Komponente gebildet werden (DE 199 45 203). Lipidpartikel haben allgemein den Vorteil, daß viele Lipide gut verträglich sind und in der Regel nicht toxisch sind. Lipidpartikel haben sich daher im allgemeinen als Wirkstoffträger gut bewährt.

Eine ganze Reihe lipophiler kosmetisch oder dermatologisch relevanter Wirkstoffe bzw. Arzneistoffe können mit Wasser spontan reagieren bzw. werden in Gegenwart von Wasser chemisch verändert. Die vorgenannten Wirkstoffformulierungen bieten in dieser Hinsicht in der Regel keinen absoluten Schutz eines in solchen Formulierungen verkapselten Wirkstoffs vor der Einwirkung von Wasser.

So erfolgt beispielsweise bereits die Herstellung von Lipidpartikeln üblicherweise unter Zusatz von Wasser, indem z.B. durch Hochdruckhomogenisation eine partikuläre Dispersion erzeugt wird. Damit kann auch der in die Lipidpartikel einzuarbeitende Wirkstoff wenigstens während der Herstellung mit Wasser in Kontakt kommen. Außerdem können sich auch in einem aus diesem Verfahren hervorgehenden Produkt immer noch Spuren von Wasser finden.

Aus diesen Gründen benötigt man auf dem Gebiet der kosmetischen und dermatologischen Anwendungen geeignete Wirkstoffträger und Zubereitungen, mit deren Hilfe die entsprechenden Wirkstoffe so formuliert werden können, daß sie während der Herstellung aber auch in der für die Anwendung fertigen Zubereitung vor der Einwirkung von Wasser geschützt sind, damit es zumindest vor der Anwendung der Zubereitung am Körper des Anwenders zu keinen Interaktionen von Wirkstoff und Wasser kommen kann.

Es besteht daher ein Bedarf nach geeigneten Wirkstoffträgern und Zubereitungen auf Lipidbasis, mit deren Hilfe lipophile Wirkstoffe im wesentlichen wasserfrei formuliert werden können, damit diese Wirkstoffe vor der Anwendung durch den Patienten vor der Einwirkung von Wasser geschützt sind. Wesentlich ist hierbei, daß der Wirkstoffträger in der Zubereitung so stabil vorliegt, daß es auch bei Raumtemperatur oder alltäglichen Umgebungstemperaturen und längerer Lagerung zu keinen Interaktionen des in dem Wirkstoffträger vorliegenden Wirkstoffs mit Wasser kommen kann.

Es ist die Aufgabe der vorliegenden Erfindung entsprechend verbesserte Wirkstoffträger und Zubereitungen bereitzustellen.

Diese Aufgabe wird gelöst durch eine Zusammensetzung mit einer wasserfreien Wirkstoffträgermatrix und wenigstens einem in dieser Wirkstoffträgermatrix eingeschlossenen lipophilen Wirkstoff, wobei die Zusammensetzung dadurch gekennzeichnet ist, daß sie
a) 25 bis 60 Gew.-% Phospholipide, wobei die Phospholipide
   i) zu 80 bis 95 Gew.-% gesättigte Phospholipide und
   ii) zu 5 bis 20 Gew.-% ungesättigte Phospholipide sind,
b) 20 bis 60 Gew.-% wenigstens ein Wachs,
c) wahlweise 5 bis 10 Gew.-% wenigstens einen Hilfsstoff und
d) 1 bis 20 Gew.-% wenigstens einen lipophilen Wirkstoff
enthält, wobei die Komponenten a) bis c) die Wirkstoffträgermatrix bilden.

Der Vorteil der Wirkstoffträgermatrix gemäß der vorliegenden Erfindung besteht darin, daß lipophile Wirkstoffe in diese Wirkstoffträgermatrix auch in hohen Konzentrationen sehr effektiv und stabil eingeschlossen werden können. Hierdurch kommen die Wirkstoffe, auch wenn sich die Wirkstoffträgermatrix mit dem darin eingeschlossenen Wirkstoff in einer wasserhaltigen Umgebung befindet, mit dem in der Umgebung enthaltenen Wasser nicht unmittelbar in Kontakt. Statt dessen werden die in der Wirkstoffträgermatrix eingeschlossenen Wirkstoffe erst dann freigesetzt, wenn sich beim Verreiben der erfindungsgemäßen Zusammensetzung in Gegenwart von Wasser auf der Haut eine Emulsion bildet. Hierdurch wird gewährleistet, daß wasserempfindliche Wirkstoffe vor der Applikation beim Anwender vor der ungewünschten Einwirkung von Wasser geschützt werden.

Der Begriff "Wirkstoffträgermatrix" ist für den Fachmann klar und eindeutig. Insbesondere ist hierunter eine Substanz zu verstehen, in die ein Wirkstoff so eingearbeitet werden kann, daß dieser Wirkstoff von der Matrix umgeben wird. Diese Substanz ist erfindungsgemäß aus bestimmten Phospholipiden, wenigstens einem Wachs und wahlweise wenigstens einem Hilfsstoff zusammengesetzt.

Die erfindungsgemäße Wirkstoffträgermatrix hat weiterhin den Vorteil, dass sie in wässerigem bzw. wasserhaltigem Milieu stabil ist, d.h. unter alltäglichen Bedingungen gewährleistet, dass der in ihr enthaltene Wirkstoff vor der Anwendung nicht mit Wasser in Kontakt kommt. Diese Stabilität weist die erfindungsgemäße Wirkstoffträgermatrix jedenfalls bei Raumtemperatur (20 bis 25 °C) auf. Vorzugsweise ist die Wirkstoffträgermatrix auch in dem Temperaturbereich von 20 bis 30°C stabil. Noch bevorzugter ist die Wirkstoffträgermatrix im Bereich von 20 bis 40°C oder gar im Bereich von 20 bis 50°C stabil. Diese Stabilität weist die erfindungsgemäße Wirkstoffträgermatrix vorzugsweise über wenigstens etwa 1 Monat auf, noch bevorzugter über wenigstens 3 Monate. Bei besonders bevorzugten Ausführungsformen weist die erfindungsgemäße Wirkstoffträgermatrix diese Stabilität über wenigstens etwa 6, 12 bzw. 36 Monate auf.

Unter der Bezeichnung "wasserfrei" bzw. "im wesentlichen wasserfrei" wird hier verstanden, dass ein mit diesem Begriff beschriebenes Material im wesentlichen kein freies Wasser enthält. Mit "im wesentlichen" ist in diesem Zusammenhang gemeint, dass nicht ausgeschlossen werden kann, dass in diesem Material trotz allem einzelne Wassermoleküle oder einige wenige Wassermoleküle vereinzelte Mikroeinschlüsse von verschwindend geringen Mengen an Wasser enthalten sind. Vorzugsweise beträgt der Gehalt an Wasser höchstens 0,5 Gew.-% bezogen auf die Wirkstoffträgermatrix. Besonders bevorzugt ist nicht mehr als 0,1 Gew.-% Wasser in der Wirkstoffträgermatrix enthalten. Noch bevorzugter ist nicht mehr als 0,01 Gew.-% Wasser in der Wirkstoffträgermatrix enthalten. Die erfindungsgemäße Wirkstoffträgermatrix hat weiterhin den Vorteil, dass sie in wässerigem bzw. wasserhaltigem Milieu stabil ist, d.h. unter alltäglichen Bedingungen gewährleistet, dass der in ihr enthaltene Wirkstoff vor der Anwendung nicht mit Wasser in Kontakt kommt. Diese Stabilität weist die erfindungsgemäße Wirkstoffträgermatrix jedenfalls bei Raumtemperatur (20 bis 25 °C) auf. Vorzugsweise ist die Wirkstoffträgermatrix auch in dem Temperaturbereich von 20 bis 30°C stabil. Noch bevorzugter ist die Wirkstoffträgermatrix im Bereich von 20 bis 40°C oder gar im Bereich von 20 bis 50°C stabil. Diese Stabilität weist die erfindungsgemäße Wirkstoffträgermatrix vorzugsweise über wenigstens etwa 1 Monat auf, noch bevorzugter über wenigstens 3 Monate. Bei besonders bevorzugten Ausführungsformen weist die erfindungsgemäße Wirkstoffträgermatrix diese Stabilität über wenigstens etwa 6, 12 bzw. 36 Monate auf.

Unter der Bezeichnung "wasserfrei" bzw. "im wesentlichen wasserfrei" wird hier verstanden, dass ein mit diesem Begriff beschriebenes Material im wesentlichen kein freies Wasser enthält. Mit "im wesentlichen" ist in diesem Zusammenhang gemeint, dass nicht ausgeschlossen werden kann, dass in diesem Material trotz allem einzelne Wassermoleküle oder einige wenige Wassermoleküle vereinzelte Mikroeinschlüsse von verschwindend geringen Mengen an Wasser enthalten sind. Vorzugsweise beträgt der Gehalt an Wasser höchstens 0,5 Gew.-% bezogen auf die Wirkstoffträgermatrix. Besonders bevorzugt ist nicht mehr als 0,1 Gew.-% Wasser in der Wirkstoffträgermatrix enthalten. Noch bevorzugter ist nicht mehr als 0,01 Gew.-% Wasser in der Wirkstoffträgermatrix enthalten.

Der Schmelzpunkt der Wirkstoffträgermatrix liegt vorzugsweise bei einer Temperatur von > 40°C. Unter dem Schmelzpunkt der Wirkstoffträgermatrix ist hierbei die Temperatur zu verstehen, die auch als Tropfpunkt bezeichnet wird, d.h. diejenige Temperatur, bei der die Wirkstoffträgermatrix zu fließen beginnt und unter definierten Umständen unter ihrem Eigengewicht von einem definierten Körper abtropft. Die Tropfpunktbestimmung kann nach DIN 51801 (Deutsche Industrienorm) mit einem Tropfpunktgerät nach Ubbelohde erfolgen.

Die Schmelzeigenschaften der Wirkstoffträgermatrix kann der Fachmann durch geeignete Auswahl der Zusammensetzung des Wachsanteils der Wirkstoffträgermatrix so einstellen, daß beim Verreiben der Wirkstoffträgermatrix auf der Haut in Gegenwart von Wasser eine Emulsion gebildet wird. Darüber hinaus können der Wirkstoffträgermatrix auch einer oder mehrere der unten aufgeführten Hilfsstoffe, wie z.B. Öle, beigefügt werden, um die gewünschten Schmelzeigenschaften einzustellen.

Ein Beispiel für eine Wirkstoffträgermatrix, die die gewünschten Schmelzeigenschaften aufweist, ist am Ende der Beschreibung angegeben.

Erfindungsgemäß ist der Wirkstoff in der Wirkstoffträgermatrix eingeschlossen. Dies ist so zu verstehen, daß der Wirkstoff bei Raumtemperatur oder alltäglichen Umgebungstemperaturen von bis zu 35, 40 oder 45°C so in der Wirkstoffträgermatrix eingebettet vorliegt, daß er von dieser vollständig umschlossen ist, so daß der Wirkstoff mit der um die Wirkstoffträgermatrix befindlichen Umgebung nicht unmittelbar in Kontakt kommt. Ein anderer Ausdruck für diesen Zustand ist, daß der Wirkstoff in der Wirkstoffträgermatrix verkapselt vorliegt.

Demnach ist die Zusammensetzung gemäß der Erfindung vorzugsweise **dadurch gekennzeichnet, daß** die wasserfreie Wirkstoffträgermatrix beim Verreiben in Gegenwart von Wasser eine Emulsion bildet. Anders ausgedrückt ist die Zusammensetzung gemäß der Erfindung vorzugsweise **dadurch gekennzeichnet, daß** die wasserfreie Wirkstoffträgermatrix, wenn sie in einer Zubereitung mit einer wasserhaltigen Zubereitungsbasis, beispielsweise in einem Gel, vorliegt, nach topischer Applikation der Zubereitung auf der Haut eine Emulsion bildet.

Hat ein Wirkstoff, der in die erfindungsgemäße Wirkstoffträgermatrix eingearbeitet wird, Einfluß auf die Schmelzeigenschaften der Zusammensetzung aus Wirkstoffträgermatrix und Wirkstoff, so gelten die oben genannten Merkmale bezüglich Schmelzeigenschaften und Fähigkeit zur Emulsionsbildung in wässrigem Milieu für die gesamte Zusammensetzung. D.h., daß in diesen Fällen die Auswahl der Zusammensetzung des Wachsanteils und wahlweise auch der Hilfsstoffe vom Fachmann so zu treffen ist, daß die Zusammensetzug aus Wirkstoffträgermatrix und Wirkstoff die geforderten Schmelzeigenschaften und die geforderte Emulgierfähigkeit aufweist.

Üblicherweise wird der Wirkstoff bei der Ausführung der Erfindung in die erfindungsgemäße Wirkstoffträgermatrix eingearbeitet, indem der Wirkstoff beispielsweise in eine zuvor zubereitete Wirkstoffträgermatrixmasse eingerührt wird. Wenn die Wirkstoffträgermatrixmasse mit dem darin eingearbeiteten Wirkstoff anschließend einem Formgebungsprozess unterworfen wird, bei dem beispielsweise Partikel erzeugt werden, ist selbstverständlich nicht auszuschließen, daß vereinzelt Wirkstoffmoleküle an der Peripherie dieser Partikel angeordnet sind, so daß diese einzelnen Wirkstoffmoleküle mit der um diese Partikel befindlichen Umgebung in Kontakt kommen können. Da der Anteil der Moleküle, die so an der Peripherie der Partikel angeordnet sind, daß sie theoretisch mit der Umgebung in Kontakt kommen könnten, im Verhältnis zu den in den Partikeln vollständig eingeschlossenen Wirkstoffmolekülen verschwindend gering ist, kann näherungsweise davon ausgegangen werden, daß bei der Ausführung der Erfindung der Wirkstoff im wesentlichen in der Wirkstoffträgermatrix eingeschlossenen vorliegt.

Gemäß der vorliegenden Erfindung enthält die Wirkstoffträgermatrix 25 bis 60 Gew.-% Phospholipide. Bei einer bevorzugten Ausführungsform der Erfindung enthält die Wirkstoffträgermatrix 40 bis 60 Gew.-% Phospholipide. Bei einer weiteren bevorzugten Ausführungsform der Erfindung enthält die Wirkstoffträgermatrix von 25 bis < 40 Gew.-% Phospholipide. Der Phospholipidanteil der Wirkstoffträgermatrix setzt sich dabei erfindungsgemäß zu 80 bis 95 Gew.-% aus gesättigten Phospholipiden und zu 5 bis 20 Gew.-% aus ungesättigten Phospholipiden zusammen.

Der Begriff "Phospholipid" ist dem Fachmann bekannt. Hiermit werden Phosphorsäuredi- und -monoester bezeichnet, die aus einem lipophilen Teil (Fettsäuren) und einer hydrophilen Kopfgruppe bestehen, wobei Fettsäuren und Kopfgruppe über einen sogenannten Abstandshalter verestert sind. Bei dem genannten Abstandshalter handelt es sich in der Regel um Glycerol.

Mit "gesättigte Phospholipide" werden hier solche Phospholipide bezeichnet, die vollständig hydriert und damit vollständig gesättigt sind. Dementsprechend sind "ungesättigte Phospholipide" solche Phospholipide, die nicht vollständig hydriert und damit auch nicht vollständig gesättigt sind. Der relativ hohe Anteil an gesättigten Phospholipiden bei der erfindungsgemäßen Wirkstoffträgermatrix führt in Kombination mit dem Wachsanteil in der Wirkstoffträgermatrix zu einer besonders hohen Stabilität von Partikeln, die aus dieser Wirkstoffträgermatrix gebildet werden.

Vorzugsweise umfassen die gesättigten Phospholipide einen Anteil von > 80 Gew.-% Phosphatidylcholin umfassen. Besonders bevorzugt beträgt der Anteil an Phosphatidylcholin > 90 Gew.-%. Die ungesättigten Phospholipide weisen vorzugsweise einen Anteil von > 75 Gew.-% Phosphatidylcholin. Noch bevorzugter beläuft sich der Anteil an ungesättigten Phospholipiden auf > 80 Gew.-% Phosphatidylcholin. Das Phosphatidylcholin dient nach dem Verreiben als Emulgator und hat den Vorteil, daß es die Emulsionsbildung besonders gut fördert.

Durch relativ hohe Anteile an Phosphatidylcholin kann die Stabilität der Partikel, die aus der erfindungsgemäßen Wirkstoffträgermatrix gebildet sind, noch zusätzlich erhöht werden. Ein hoher Phosphatidylcholingehalt von > 75 Gew.-%, > 80 Gew.-% oder gar > 90 Gew.-% führt zu Partikeln, die in wasserhaltigen Zubereitungen, z.B. in Hydrogelen, besonders stabil sind.

Gemäß der vorliegenden Erfindung enthält die Wirkstoffträgermatrix 20 bis 60 Gew.-% wenigstens ein Wachs. Bei einer bevorzugten Ausführungsform der Erfindung enthält die Wirkstoffträgermatrix 20 bis 40 Gew.-% Wachs. Bei einer weiteren bevorzugten Ausführungsform der Erfindung enthält die Wirkstoffträgermatrix 30 bis 60 Gew.-% Wachs. Bei einer weiteren bevorzugten Ausführungsform der Erfindung enthält die Wirkstoffträgermatrix von > 40 bis 60 Gew.-% Wachs. Unter dem Begriff "Wachs" werden hier alle pflanzlichen, tierischen, mineralischen oder synthetischen Wachse und Wachsester verstanden. Unter "Wachsester" versteht der Fachmann die Ester langkettiger Wachssäuren, die wenigstens 22 Kohlenstoffatome aufweisen, mit langkettigen Wachsalkoholen, die etwa 24 - 36 Kohlenstoffatome aufweisen, Triterpenalkoholen oder Steroidalkoholen, sowie Gemische verschiedener Wachsester. Die pflanzlichen, tierischen, mineralischen oder synthetischen Wachse können aus Wachsestern bestehen oder nur einen Anteil von Wachsestern aufweisen bzw. Wachsester als Hauptkomponente aufweisen. Die pflanzlichen, tierischen, mineralischen oder synthetischen Wachse umfassen unabhängig davon, ob sie Wachsester enthalten, auch solche Stoffe oder Stoffgemische, die bei 20°C knetbar, fest bis brüchig hart sind, eine grobe bis feinkristalline Struktur aufweisen und farblich durchscheinend bis opak, aber nicht glasartig sind, und wenn diese Stoffe bzw. das Stoffgemische bei über 40°C ohne Zersetzung schmelzen, schon wenig oberhalb des Schmelzpunktes niedrigviskos und nicht fadenziehend sind, eine stark temperaturabhängige Konsistenz und Löslichkeit aufweisen sowie unter leichtem Druck polierbar sind.

Zu den Wachsen, die bei der vorliegenden Erfindung zur Anwendung kommen können, zählen insbesondere: Weißtannennadelwachs, Akazienblattwachs, Bienenwachs, hydrolysiertes Bienenwachs, oxidiertes Bienenwachs, PEG-6 Bienenwachs; PEG-8 Bienenwachs, PEG-12 Bienenwachs, PEG-20 Bienenwachs, synthetisches Bienenwachs, Carnaubawachs und saures Carnaubawachs, hydrolysiertes Carnaubawachs, PEG-12 Carnaubawachs, synthetisches Carnaubawachs, Ceresin, Cetylester, Zistrosenblütenwachs (Labdanum), Pomeranzenblütenwachs (Bitterorangenblütenwachs), Orangenblütenwachs, Orangenschalenwachs, Ginsterblütenwachs, Mehlblumenwachs, Schildlauswachs, Candelillawachs, hydrolysiertes Candelillawachs, synthetisches Candelillawachs, Strohblumenwachs (Currykrautwachs), Japanwachs, hydrogeniertes Japanwachs, synthetisches Japanwachs, Jojobabutter, Jojobawachs, Jojobaester, hydrogeniertes Jojobawachs, geschwefeltes Jojobawachs, mikrokristallines Wachs, hydrogeniertes mikrokristallines Wachs, oxidiertes mikrokristallines Wachs, oxidiertes mikrokristallines Wachs - Kaliumsalz, Reiskleienwachs, hydrogeniertes Reiskleienwachs, Lilienblütenwachs, Jasminblütenwachs, Lanolin (Wollwachs), Lavendelblütenwachs, Hennawachs, Nerzwachs, Montanwachs und saures Montanwachs, Wachsmyrtenfruchtwachs, Basilikumwachs (indisch), Ouricurywachs, Ozokerit, Palmkernwachs, Paraffin, Avocadowachs, Mastixblattwachs, Nachthyazinthenblütenwachs, Apfelschalenwachs, Johannisbeerenknospenwachs, Rosenblütenwachs, Rispenrosenblütenwachs, Muskatellersalbeiwachs, Schellack, Gerstentreberwachs, Halfagraswachs, synthetische Wachse, wie z.B. Polyethylenwachse, Polyethylenglykolwachse, Polyethylenglykolesterwachse, wachsartige Silicone, Traubenblattwachs, Zuckerrohrwachs, Walrat, Bürzeldrüsenfett.

Über die Zusammensetzung des Wachsanteils können die Schmelzeigenschaften der Wirkstoffträgermatrix beeinflußt werden. Wird ein Wachs mit niedrigem Schmelzpunkt in die Wirkstoffträgermatrix eingearbeitet, so wird die Wirkstoffträgermatrix insgesamt weicher und schmilzt bei niedrigeren Temperaturen. Wenn ein Wachs mit höherem Schmelzpunkt verwendet wird, so erhält man eine härtere Wirkstoffträgermatrix. Besonders bevorzugt sind Ausführungsformen, bei denen der Wachsanteil aus einem oder mehreren unter Japanwachs, Jojobawachs, Bienenwachs oder Candililawachs besteht.

Wahlweise kann die Wirkstoffträgermatrix bei der vorliegenden Erfindung 5 bis 10 Gew.-% wenigstens einen Hilfsstoff enthalten. Der Begriff "Hilfsstoff" ist dem Fachmann im Zusammenhang mit Wirkstoffträgermatrices bzw. kosmetischen oder dermatologischen Zusammensetzungen und Zubereitungen bekannt. Insbesondere fallen unter diesen Begriff hier solche Zusatzstoffe, die auf die physikalischen Eigenschaften der Zusammensetzung und deren Stabilität einwirken und/oder der Konservierung der Zubereitung dienen. Beispiele für solche Hilfs- oder Zusatzstoffe sind Tenside, Emulgatoren, Detergenzien, Kieselsäure, Öle, Fette, Alkohole, Gelbildner, Gelatine, anorganische Zusätze, Konservierungsmittel, Bakterizide und Keimhemmer, Verdicker, Elektrolyte oder Komplexbildner.

Zu den Ölen und Fetten, die bei der vorliegenden Erfindung als Hilfsstoff zur Anwendung kommen können, zählen: Rizinusöl (auch hydriert, teilhydriert und/oder acetyliert), Kiwisamenöl, Affenbrotbaumöl, Lichtnussbaumöl, Knoblauchöl, Cashewsamenöl, Engelwurzöl, Erdnussöl, Klettensamenöl, Arganöl, Stachelmohnöl (Argemonöl), Haferkornöl, Dattelsamenöl, Galambutter, Madhucabutter, Paranussöl, Annattosamenöl, Borretschsamenöl, Hirnlipide, Rapssamenöl, Broccolisamenöl, Büffelfett, Butter, Sheabutter und Sheabutteröl, C8-C12 Fettsäuretriglyceride, C10-C18 Fettsäuretriglyceride, C12-C18 Fettsäuretriglyceride, Ringelblumensamenöl, Kapkastanienöl, Tamanuöl (Calophyllumöl), Kameliensamenöl, Teesamenöl, Nangainussöl (Kenarinussöl), Hanfsamenöl, Canolaöl, Caprylsäure/Caprinsäure/Laurinsäure-Triglyceride, Caprylsäure/Caprinsäure/Linolsäure-Triglyceride, Caprylsäure/Caprinsäure/Myristinsäure/Stearinsäure-Triglyceride, Caprylsäure/Caprinsäure/Stearinsäure-Triglyceride, Caprylsäure/Caprinsäure-Triglyceride, Laurinsäure/Palmitinsäure/Ölsäure-Triglyceride, Ölsäure/Linolsäure-Triglyceride, Rizinolsäure/Capronsäure/Caprylsäure/Caprinsäure-Triglyceride, Ölsäure/Palmitinsäure/Laurinsäure/Myristinsäure/Linolsäure-Triglyceride, Papayasamenöl, Chaulmoograsamenöl, Gorlisamenöl, Distelsamenöl, Pekannussöl, Caryocarfruchtöl, Caryocarsamenöl, Rizinusölbenzoat, Kephaline, Quinoasamenöl, Schatullenfarnöl (chinesisch), C10-C40 Isoalkylfettsäuretriglyceride, Wassermelonensamenöl, Kokosnussöl, Dorschleber-/Nerztalgtriglyceride, Dorschleberöl (Lebertran), Kaffeesamenöl, Hiobstränensamenöl, Haselnussöl, Krambesamenöl, Gurkenkernöl, Kürbiskernöl, Karottensamenöl, Hirschtalg/Hirschfett, Samenöl des Wegerichblättrigen Natternkopfs, Palmkernöl, Palmöl, Emuöl, epoxidiertes Sojaöl, Kohlpalmenfruchtöl, Fischleberöl, Fischöl, Kokumbutter, Avelanaöl, Glyceryltriacetylhydroxystearat, Glyceryltriacetylricinoleat, trihydrogeniertes Glycerylrosinat, Sojalipide, Sojaöl, Glycolipide, Glycosphingolipide, Ziegenbutter, Ziegenfett, Baumwollsamenöl, Blutregenalgenöl, Sonnenblumenkernöl, Sanddornöl, Pferdefett, Placentalipide, Mangobutter (afrikanisch), Mangosamenöl, Färberwaidsamenöl (Deutscher Indigo), i-somerisiertes Palmöl, Walnussöl, Schweineschmalz/Schweinefett, Wiesenschaumkrautsamenöl, Leinsamenöl, Samenöl des Echten Steinsamen, Schwammkürbissamenöl, Lupinensamenöl, Bocksdornsamenöl, Macadamianussöl, Mamushi Schlangenöl, Murmeltieröl, Knochenmarklipide, Teebaumöl, Neemsamenöl (Niemsamenöl), Melissensamenöl, Menhadenöl, Milchlipide, Nerzöl, Nonisamenöl, Behensamenöl (Moringaöl), Mortierellaöl, Öl gewonnen durch *Mucor circinelloides*, Perlmuttlipide, Klauenöl/Knochenöl, Lotusblumenöl, Schwarzkümmelöl, Kieselalgenöl, Bacabapalmenfruchtöl, Batauapalmenfruchtöl, Nachtkerzenöl, Olivenfruchtöl, Olivenschalenöl, Oleostearin, Rindernetzlipide, Granatbarschöl (Orange Roughy), Babassupalmensamenöl, Babassuöl, Babassukernöl, Reiskeimöl, Reiskleienöl, Straußenöl, oxidiertes Maisöl, oxidiertes Haselnussöl, Moosbeerensamenöl, Mohnsamenöl, Schlafmohnsamenöl, Mobolapflaumensamenöl, Passionsblumensamenöl, Perillasamenöl, Avocadobutter, Avocadoöl, Flachssamenöl, Korea-Kiefernsamenöl, Bonsai-Kiefernsamenöl, Mädchenkiefernsamenöl, Pfeffersamenöl, Pistazienkernöl, Plazentalipide, Sacha-Inchi-Öl, Pongamsamenöl, Bittermandelkernöl, Mandelöl, Aprikosenkernöl, Süßkirschensamenöl, Sauerkirschenkernöl, Pflaumenkernöl, Wildpflaumenkernöl, Pfirsichkernöl, Granatapfelkernöl, Ilombanussöl, Apfelöl, Apfelsamenöl, Kaninchenfett, Johannisbeerensamenöl, Mongongosamenöl, Rizinussamenöl, Hagebuttenfruchtöl, Hagebuttenkernöl, Moschusrosensamenöl, Wildrosensamenöl, Moltebeerensamenöl, Andenbeerensamenöl, Himbeerensamenöl, Lachsöl, Chiasamenöl, Sandelholzsamenöl, Pfefferbaumöl (peruanisch), Mankettikernöl, Lackbaumsamenöl, Marulabaumsamenöl, Sesamsamenöl, Hailipide, Haileberöl, Salbutter, Seidenraupenöl, Seidenraupenlipide, Hautlipide, Tomatenfruchtöl, Tomatensamenöl, Sojabohnenglyceride, Sphingolipide, Rückenmarklipide, Stinkbaumsamenöl, Tallöl, Talg, Agaröl (pazifisch), Kakaobutter, Nusseibensamenöl, Triarachidin, Tribehenin, Tricaprin, Tricaprylin, Buschtabaköl (australisch), Trierucin, Triethylhexanoin, Triheptanoin, Triheptylundecanoin, Trihydroxymethoxystearin, Trihydroxystearin, Trüsononanoin, Triisopalmitin, Triisostearin, Trilaurin, Trilinolein, Trilinolenin, Trimyristin, Triolein, Tripalmitin, Tripalmitolein, Triricinolein, Trisebacin, Tristearin, Triundecanoin, Weizenkleienlipide, Weizenkeimöl, Schildkrötenöl, Kranbeerensamenöl (Moosbeerensamenöl), Blaubeerensamenöl, Preiselbeerensamenöl, Vanillefruchtöl, Vateriatalg (Vateriabutter), Pflanzenöl, Virolafett (Virolanussöl), Traubenkernöl, Maiskeimöl, Maisöl sowie deren hydrogenierte und/oder teilhydrogenierte Derivate.

Gemäß der vorliegenden Erfindung enthält die Zusammensetzung 1 bis 20 Gew.-% wenigstens einen lipophilen Wirkstoff, der in der Wirkstoffträgermatrix eingeschlossen vorliegt. Die Wirkstoffträgermatrix gemäß der vorliegenden Erfindung hat den Vorteil, daß sie besonders große Mengen Wirkstoff in sich aufnehmen kann. Bei bevorzugten Ausführungsformen ist der Wirkstoffanteil daher im Bereich von 5 bis 20 Gew.-%. Liegen mehrere Wirkstoffe vor, so macht die Summe der Wirkstoffe 1 bis 20 Gew.-%, vorzugsweise 5 bis 20 Gew.-% aus. Der Begriff "Wirkstoff" ist dem Fachmann bekannt und ist im Zusammenhang mit dieser Erfindung möglichst breit zu verstehen. Dieser Begriff umfaßt in diesem breiten Sinne alle Stoffe, die bei der Anwendung an einem Lebewesen eine physiologische, antibiotische oder protektive Wirkung entfalten können. Im Zusammenhang mit der erfindungsgemäßen kosmetischen oder dermatologischen Zubereitung handelt es sich entsprechend vorzugsweise um einen in irgendeiner Weise kosmetisch oder dermatologisch relevanten Wirkstoff, d.h. um einen Stoff, der in kosmetischer oder dermatologischer Hinsicht eine positive Wirkung haben kann.

Für den Fachmann ist auch klar, welche Wirkstoffe als lipophil zu bezeichnen sind. Insbesondere ist ein Wirkstoff gemäß der vorliegenden Erfindung dann als lipophil zu bezeichnen, wenn dieser Wirkstoff sich gut in Fetten und Ölen, beispielsweise in den oben aufgezählten, bei der Erfindung verwendbaren Fetten und Ölen, lösen läßt.

Der Wirkstoff kann, ohne daß die Erfindung hierauf in irgendeiner Weise beschränkt ist, beispielsweise ausgewählt sein unter Corticoiden, Fungiziden, Antibiotika, nichtsteroidalen antiinflammatorisch und antientzündlich wirkenden Substanzen, Antirheumatika, Anästhetika, Antiphlogistika sowie schmerzstillenden oder -lindernden Substanzen, Vitaminen, essentiellen Fettsäuren, Desodorantien bzw. Antitranspirantien, Insektenrepellentien, UVA- bzw. UVB-Strahlung absorbierenden Stoffen, Farbstoffen und Pigmenten.

Vorzugsweise ist der wenigstens eine lipophile Wirkstoff ausgewählt unter einem Stoff der Vitamin A-Gruppe oder einem Derivat davon. Besonders bevorzugt enthält die erfindungsgemäße Zusammensetzung Retinol (Vitamin A₁).

Die kosmetische Relevanz von Vitamin A besteht in dessen Funktion als Bioregulator bei der Bildung von Keratinozyten und ihrer Differenzierung. Topisch appliziertes Vitamin A induziert eine vermehrte Zellteilung in der Basalschicht und führt damit zu einer Verdickung der Epidermis und zu einer Verbesserung von deren Barriereeigenschaften [D. F. Counts et al., J. Soc. Cosmet. Chem. 39, 235-240, 1988]. Tägliches Auftragen einer Emulsion mit einer geringen Konzentration von 1000 - 5000 IE Retinol pro ml [49,5% = 1.650.000 IE/g (ml)] führt innerhalb von wenigen Tagen zu einer Verdickung der Epidermis um 50% bzw. 70% gegenüber der Anwendung eines Placebo. Weiterhin steigert Vitamin A die Bildung von Kollagen und übt regulierende Wirkung auf den Keratinisierungsprozeß der Epidermis aus. Retinol ist damit besonders interessant für Präparate für die alternde Haut, bei der häufig eine besonders dünne Epidermis mit einer vermehrten Faltenanzahl vorliegt ("Zigarettenpapierhaut") [F. Reiss und R. M. Campbell, Dermatologica 1208, 121-128, 1954]. Ältere Haut ist oft auch gekennzeichnet durch einen erhöhten transepidermalen Wasserverlust, welcher die Haut austrocknet und zu Brüchigkeit und Rissen führt. Vitamin A wirkt der altersbedingten Haut regulierend entgegen, indem es deren Aussehen speziell durch eine Zunahme der Elastizität und der Dicke der Epidermis verbessert.

Antiphlogistische, analgetische und cytoprotektive Eigenschaften wie auch die antioxidative Wirkung machen die Liponsäure zu einem interessanten Wirkstoff für Pharmazie, Kosmetik, Ernährungswissenschaft und angrenzende Gebiete. Alpha-Liponsäure kann eine so große Menge freier Radikale unschädlich machen wie kein anderes Antioxidans. Dabei ist einer der Kerneffekte von Alpha-Liponsäure die Regeneration anderer, verbrauchter Antioxidantien, wie Vitamin C und E, Coenzym Q10 und Glutathion. Vorzugsweise ist daher der wenigstens eine lipophile Wirkstoff Liponsäure oder ein Derivat davon.

Die Stoffe der Vitamin A-Gruppe sind relativ instabil. Insbesondere das Retinol wird in Gegenwart von Wasser bereits bei Raumtemperatur recht schnell inaktiviert. Auch die Liponsäure ist in Gegenwart von Wasser bereits bei Raumtemperatur recht instabil und wird schnell abgebaut. Außerdem weist Liponsäure in ihrer aktiven Form (d.h. als Antioxidans) einen charakteristischen Schwefelgeruch auf und kann deshalb nur sehr bedingt in kosmetische Formulierungen eingearbeitet werden.

Der Vorteil der Wirkstoffträgermatrix gemäß der vorliegenden Erfindung im Hinblick auf gerade diese Wirkstoffe besteht dementsprechend darin, daß diese Wirkstoffe in der Wirkstoffträgermatrix so eingeschlossen vorliegen, daß sie, selbst wenn sich die sie umgebende Wirkstoffträgermatrix in einer wasserhaltigen Umgebung befindet, mit dem in der Umgebung enthaltenen Wasser nicht unmittelbar in Kontakt kommen und so vor der ungewünschten Einwirkung von Wasser geschützt werden. Im Fall der Liponsäure ist dies besonders vorteilhaft, da diese hierdurch zunächst in ihrer geruchsärmeren inaktiven Form in die Wirkstoffträgermatrix eingearbeitet werden kann, wobei diese inaktive Form dann beim Verreiben der Wirkstoffträgermatrix auf der Haut, mit Wasser einer wasserhaltigen Zubereitungsbasis, in der die Wirkstoffträgermatrix vorlag, in Kontakt kommen und dadurch aktiviert werden kann.

Je nach Anwendung und Wirkstoff ist es bei bestimmten Ausführungsformen bevorzugt, wenn die Zusammensetzung bzw. Zubereitung nur einen Wirkstoff enthält. Bei alternativen Ausführungsformen ist es jedoch bevorzugt, wenn die Zusammensetzung bzw. Zubereitung mehrere Wirkstoffe umfaßt. Bei einer speziell bevorzugten Ausführungsform enthält die erfindungsgemäße Zusammensetzung beispielsweise sowohl einen Stoff der Vitamin A-Gruppe als auch Liponsäure. Der oder die Wirkstoffe können entweder alle in der Wirkstoffträgermatrix eingeschlossen vorliegen oder es können einer oder mehrere lipophile Wirkstoffe in der Wirkstoffträgermatrix und darüber hinaus einer oder mehrere hydrophile Wirkstoffe in der wasserhaltigen Zubereitungsbasis vorliegen.

Im Sinne der klaren Abgrenzung der einzelnen Bestandteile der erfindungsgemäßen Zusammensetzung soll an dieser Stelle noch angemerkt werden, daß ein Stoff, der in dieser Zusammensetzung enthalten ist und der unter die Definition von Phospholipid fällt, ausschließlich einen Beitrag zu dem Phospholipidanteil der Zusammensetzung leistet, selbst wenn dieser Stoff auch unter die Definition einer anderen Komponente der erfindungsgemäßen Zusammensetzung fällt. Das gleiche gilt entsprechend für einen Stoff, der in dieser Zusammensetzung enthalten ist und der unter die Definition von Wachs im Sinne dieser Erfindung fällt, es sei denn dieser Stoff fällt bereits unter die Definition von Phospholipid. Und das gleiche gilt entsprechend für einen Stoff, der in dieser Zusammensetzung enthalten ist und der unter die Definition von Wirkstoff im Sinne dieser Erfindung fällt, es sei denn dieser Stoff fällt bereits unter die Definition von Phospholipid oder Wachs im Sinne dieser Erfindung.

Selbstverständlich handelt es sich bei allen Komponenten der erfindungsgemäßen Zusammensetzung um kosmetisch oder dermatologisch verträgliche Stoffe. Ein Stoff ist im Sinne dieser Erfindung kosmetisch oder dermatologisch verträglich, wenn er nicht toxisch ist und bei der Mehrzahl der potentiellen Anwender topisch anwendbar ist, ohne daß es bei dem Anwender spontan oder nach einer Weile zu einer ungewünschten physiologischen Reaktion, wie z.B. einer Rötung oder der Ausbildung eines Juckreizes kommt.

Bei einer Ausführungsform der Erfindung liegt die Wirkstoffträgermatrix in Form von Partikeln vor. Vorzugsweise beträgt der mittlere Durchmesser der Partikel von 0,1 bis 3 mm. Besonders bevorzugt sind relativ kleine Partikel mit einem mittleren Durchmesser von 0,1 bis 1 mm, da solche Partikel schneller schmelzen können und sich daher etwas besser verreiben lassen. Alternativ sind auch relativ große Partikel mit einem mittleren Durchmesser von 1 bis 3 mm besonders bevorzugt, da solche Partikel ein vorteilhaft großes Verhältnis von Volumen zu Oberfläche haben und daher anteilsmäßig deutlich mehr Wirkstoffmoleküle in der Wirkstoffträgermatrix vollständig eingeschlossen sind (siehe oben). Eine weitere alternative Ausführungsform stellt einen optimalen Kompromiss zwischen den beiden zuvor erwähnten vorteilhaften Bereichen dar und ist durch Partikel mit einem mittleren Durchmesser von 0,5 bis 1,5 mm gekennzeichnet.

Neben der oben diskutierten Zusammensetzung aus Wirkstoffträgermatrix und Wirkstoff umfaßt die vorliegende Erfindung auch eine kosmetische oder dermatologische Zubereitung, die eine wasserhaltige Zubereitungsbasis und eine in der Zubereitungsbasis vorliegende erfindungsgemäße Wirkstoffzusammensetzung, wie sie oben diskutiert wird, umfaßt. Vorzugsweise ist die Wirkstoffträgermatrix in der Zubereitungsbasis suspendiert. Bei der erfindungsgemäßen Zubereitung liegt der wenigstens eine Wirkstoff im Inneren der Wirkstoffträgermatrix so eingeschlossen vor, daß vor der kosmetischen oder dermatologischen Anwendung der Zubereitung kein unmittelbarer Kontakt des Wirkstoffs mit der Zubereitungsbasis besteht.

Die kosmetische oder dermatologische Zubereitung gemäß der vorliegenden Erfindung ist für die topische Anwendung bestimmt. Wird diese Zubereitung bei der topischen Anwendung beispielsweise auf der Haut verstrichen oder verrieben, so vermischt sich der Wasseranteil der wasserhaltigen Zubereitungsbasis mit der aufgrund der Wärme der Haut oder der beim Verreiben zusätzlich entstehenden Wärme allmählich schmelzenden Wirkstoffträgermatrix, wodurch die Wirkstoffträgermatrix emulgiert wird. Die wasserfreie Wirkstoffträgermatrix bildet nach dem Auftragen einer diese Wirkstoffträgermatrix enthaltenden Zubereitung gemäß der vorliegenden Erfindung im Gegensatz zu herkömmlichen Zubereitungen mit Lipidpartikeln auf der Haut mit der wasserhaltigen Zubereitungsbasis eine Emulsion.

Demnach ist die kosmetische oder dermatologische Zubereitung gemäß der Erfindung vorzugsweise **dadurch gekennzeichnet, daß** die wasserfreie Wirkstoffträgermatrix nach dem Verreiben der Wirkstoffträgermatrix in der wasserhaltigen Zubereitungsbasis eine Emulsion bildet. Anders ausgedrückt ist die kosmetische oder dermatologische Zubereitung gemäß der Erfindung vorzugsweise **dadurch gekennzeichnet, daß** die wasserfreie Wirkstoffträgermatrix in der wasserhaltigen Zubereitungsbasis nach topischer Applikation der Zubereitung auf der Haut eine Emulsion bildet.

Vorzugsweise handelt es sich bei der wasserhaltigen Zubereitungsbasis um ein Gel, d.h. um ein feindisperses System aus mindestens einer festen und einer flüssigen Phase, wobei die feste Phase ein schwammartiges, dreidimensionales Netzwerk bildet, dessen Poren durch eine Flüssigkeit ausgefüllt sind, und wobei sich beide Phasen vollständig durchdringen. Die flüssige Phase enthält wenigstens einen Anteil an Wasser und besteht vorzugsweise sogar zu einem Großteil aus Wasser. Eine besonders geeignete Formulierung der erfindungsgemäßen Zusammensetzung ist ein einfaches Hydrogel, welches aus Wasser und einem Verdicker (Gelbildner) besteht.

Gerade im Falle einer Gelformulierung gemäß der vorliegenden Erfindung bildet die in dem Gel vorliegende wasserfreie Wirkstoffträgermatrix nach topischer Applikation auf der Haut mit den Bestandteilen des Gels eine besonders vorteilhafte Emulsion aus.

Mit der Wirkstoffträgermatrix gemäß der vorliegenden Erfindung ist es, wie oben bereits ausgeführt wurde, nun möglich, lipophile Wirkstoffe, wie beispielsweise Retinol und/oder Liponsäure, vor Wasser sicher geschützt und stabil in der wasserfreien Wirkstoffmatrix einzuschließen.

Eine bevorzugte Zusammensetzung gemäß der vorliegenden Erfindung ist dadurch gekennzeichnet ist, daß sie a) 25 bis 60 Gew.-% Phospholipide, b) 30 bis 60 Gew.-% Wachs, c) wahlweise 5 bis 10 Gew.-% wenigstens einen Hilfsstoff und d) 1 bis 20 Gew.-% wenigstens einen lipophilen Wirkstoff enthält, wobei die Komponenten a) bis c) die Wirkstoffträgermatrix bilden.

Eine weitere bevorzugte Zusammensetzung gemäß der vorliegenden Erfindung ist dadurch gekennzeichnet ist, daß sie a) 40 bis 60 Gew.-% Phospholipide, b) 20 bis 40 Gew.-% Wachs, c) wahlweise 5 bis 10 Gew.-% wenigstens einen Hilfsstoff und d) 1 bis 20 Gew.-% wenigstens einen lipophilen Wirkstoff enthält, wobei die Komponenten a) bis c) die Wirkstoffträgermatrix bilden.

Eine weitere bevorzugte Zusammensetzung gemäß der vorliegenden Erfindung ist dadurch gekennzeichnet ist, daß sie a) von 25 bis < 40 Gew.-% Phospholipide, b) von > 40 bis 60 Gew.-% Wachs, c) wahlweise 5 bis 10 Gew.-% wenigstens einen Hilfsstoff und d) 1 bis 20 Gew.-% wenigstens einen lipophilen Wirkstoff enthält, wobei die Komponenten a) bis c) die Wirkstoffträgermatrix bilden.

Der Phospholipidanteil der Wirkstoffträgermatrix setzt sich bei den in den drei vorherigen Absätzen genannten Zusammensetzungen jeweils zu 80 bis 95 Gew.-% aus gesättigten Phospholipiden und zu 5 bis 20 Gew.-% aus ungesättigten Phospholipiden zusammen.

Die vorliegende Erfindung betrifft außer der erfindungsgemäßen Zusammensetzung aus Wirkstoffträgermatrix und Wirkstoff bzw. einer erfindungsgemäßen Zubereitung, die eine solche Zusammensetzung aus Wirkstoffträgermatrix und Wirkstoff enthält, außerdem auch ein Verfahren zu deren Herstellung, wobei man bei diesem Verfahren:
a) die Wachskomponente der Wirkstoffträgermatrix gemeinsam mit den ungesättigten Phospholipiden der Wirkstoffträgermatrix schmilzt,
b) die gesättigten Phospholipide der Wirkstoffträgermatrix mit der Schmelze aus a) vermischt und
c) den wenigstens einen Wirkstoff in das Gemisch aus b) einarbeitet.

Besonders bevorzugt erzeugt man in einem weiteren anschließenden Verfahrensschritt aus der oben beschriebenen Masse aus Wirkstoffträgermatrix und darin eingearbeitetem Wirkstoff nach bekannten Verfahren Partikel oder Granulate.

Für Zwecke der ursprünglichen Offenbarung wird darauf hingewiesen, daß sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Weitere Merkmale, Merkmalskombinationen und Vorteile der vorliegenden Erfindung werden aus den folgenden nicht beschränkenden Beispielen ersichtlich.

### Beispiele:

### 1. Beispiel 1

Japanwachs und ungesättigte Phospholipide mit einem Gehalt von 80 Gew.-% an Phosphatidylcholin (PL80) werden zusammen mit dem Hilfsstoff Pentandiol aufgeschmolzen. Danach werden hydrierte Phospholipide mit einem Gehalt 90 Gew.-% an Phosphatidylcholin (PL90H) zugegeben. Aus dem hieraus hervorgehenden Gemisch wird eine klare Schmelze erzeugt. Liegt eine klare Schmelze vor, wird der Wirkstoff Liponsäure hinzugefügt. Die Schmelze wird dann warm weiterverarbeitet.

Zu den geeigneten Verfahren zur Herstellung der Partikel zählen Sprüherstarrungs- (Prilling-Verfahren) und Sprühtrocknungsverfahren unter Verwendung eines *Rotating Disc Atomizers* sowie das von der Firma Brace GmbH in Alzenau etablierte Mikrokugelverfahren und die von der Firma Genialab GmbH in Braunschweig etablierte Jet-Cutter-Technologie.

Die aus diesem Verfahren hervorgehenden emulgierenden Lipidpartikel (Wirkstoffträgermatrix), in denen der Wirkstoff Liponsäure eingeschlossen ist, haben die folgende Zusammensetzung:

| | |
|---|---|
| 25,0 Gew.-% | PL 90 H |
| 30,0 Gew.-% | Japanwachs |
| 19,0 Gew.-% | Jojobawachs (70) |
| 14,0 Gew.-% | Jojobawachs (60) |
| 7,5 Gew.-% | Pentandiol |
| 2,0 Gew.-% | Liponsäure |
| 2,5 Gew.-% | PL 80 |

### 2. Beispiel 2

Japanwachs und ungesättigte Phospholipide mit einem Gehalt von > 85 Gew.-% an Phosphatidylcholin (PL85) werden zusammen mit dem Hilfsstoff Pentylenglycol aufgeschmolzen. Danach werden hydrierte Phospholipide mit einem Gehalt> 90 Gew.-% an Phosphatidylcholin (PL90H) zugegeben. Aus dem hieraus hervorgehenden Gemisch wird eine klare Schmelze erzeugt. Liegt eine klare Schmelze vor, werden die Wirkstoffe Retinol und Liponsäure hinzugefügt. Die Schmelze wird dann warm weiterverarbeitet.

Zu den geeigneten Verfahren zur Herstellung der Partikel zählen Sprüherstarrungs- (Prilling-Verfahren) und Sprühtrocknungsverfahren unter Verwendung eines *Rotating Disc Atomizers* sowie das von der Firma Brace GmbH in Alzenau etablierte Mikrokugelverfahren und die von der Firma Genialab GmbH in Braunschweig etablierte Jet-Cutter-Technologie.

Die aus diesem Verfahren hervorgehenden emulgierenden Lipidpartikel (Wirkstoffträgermatrix), in denen die Wirkstoffe Retinol und Liponsäure eingeschlossen sind, haben die folgende Zusammensetzung:

| | |
|---|---|
| 46,5 Gew.-% | PL 90 H |
| 36,0 Gew.-% | Japanwachs |
| 6,0 Gew.-% | Pentylenglycol |
| 5,0 Gew.-% | Retinol 50C |
| 2,5 Gew.-% | Liponsäure |
| 4,0 Gew.-% | PL 85 |

## Patentansprüche

1. Kosmetische oder dermatologische Zusammensetzung mit einer wasserfreien Wirkstoffträgermatrix und wenigstens einem in dieser Wirkstoffträgermatrix verkapselten lipophilen Wirkstoff, wobei die Zusammensetzung **dadurch gekennzeichnet ist, daß** sie
a) 25 bis 60 Gew.-% Phospholipide, wobei
die Phospholipide
i) zu 80 bis 95 Gew.-% gesättigte Phospholipide und
ii) zu 5 bis 20 Gew.-% ungesättigte Phospholipide sind,
b) 20 bis 60 Gew.-% wenigstens ein Wachs,
c) wahlweise 5 bis 10 Gew.-% wenigstens einen Hilfsstoff und
d) 1 bis 20 Gew.-% wenigstens einen lipophilen Wirkstoff
enthält, wobei die Komponenten a) bis c) die Wirkstoffträgermatrix bilden.

2. Zusammensetzung nach Anspruch 1, **dadurch gekennzeichnet, daß** die gesättigten Phospholipide einen Anteil von > 80 Gew.-% Phosphatidylcholin umfassen.

3. Zusammensetzung nach einem der Ansprüche 1 und 2, **dadurch gekennzeichnet, daß** die ungesättigten Phospholipide einen Anteil von > 75 Gew.-% Phosphatidylcholin umfassen.

4. Zusammensetzung nach einem der Ansprüche 1 bis 3, **dadurch gekennzeichnet, daß** die Wirkstoffträgermatrix bei der topischen Applikation auf der Haut in der Gegenwart von Wasser eine Emulsion bildet.

5. Zusammensetzung nach Anspruch 4, **dadurch gekennzeichnet, daß** die Wirkstoffträgermatrix wenigstens ein Wachs unter Japanwachs, Jojobawachs, Bienenwachs, Candililawachs, Shea-Butter und Cacaobutter umfaßt.

6. Zusammensetzung nach einem der Ansprüche 1 bis 5, **dadurch gekennzeichnet, daß** die Wirkstoffträgermatrix in Form von Partikeln vorliegt.

7. Zusammensetzung nach Anspruch 6, **dadurch gekennzeichnet, daß** der mittlere Durchmesser der Partikel im Bereich von 0,1 bis 3 mm liegt.

8. Zusammensetzung nach einem der Ansprüche 1 bis 7, **dadurch gekennzeichnet, daß** der wenigstens eine Wirkstoff ausgewählt ist unter einem Stoff der Vitamin A-Gruppe oder Liponsäure und deren Derivaten oder Gemischen davon.

9. Kosmetische oder dermatologische Zubereitung umfassend
a) eine wasserhaltige Zubereitungsbasis,
b) eine in der Zubereitungsbasis suspendierte Zusammensetzung nach einem der Ansprüche 1 bis 8,
wobei der wenigstens eine Wirkstoff im Inneren der Wirkstoffträgermatrix so eingekapselt vorliegt, daß vor der kosmetischen oder dermatologischen Anwendung der Zubereitung kein unmittelbarer Kontakt des Wirkstoffs mit der Zubereitungsbasis besteht und wobei die wasserfreie Wirkstoffträgermatrix mit der wasserhaltigen Zubereitungsbasis bei der topischen Applikation auf der Haut eine Emulsion bildet.

10. Zubereitung nach Anspruch 9, **dadurch gekennzeichnet, daß** die Zubereitung eine Gelformulierung ist, wobei die wasserfreie Wirkstoffträgermatrix mit der Gelformulierung bei der topischen Applikation auf der Haut eine Emulsion bildet.

11. Verfahren zur Herstellung einer Zusammensetzung nach einem der Ansprüche 1 bis 8 oder einer Zubereitung nach einem der Ansprüche 9 oder 10, bei dem man
a) die Wachskomponente der Wirkstoffträgermatrix gemeinsam mit den ungesättigten Phospholipiden der Wirkstoffträgermatrix schmilzt,
b) die gesättigten Phospholipide der Wirkstoffträgermatrix mit der Schmelze aus a) vermischt und
c) den wenigstens einen Wirkstoff in das Gemisch aus b) einarbeitet.

12. Verfahren nach Anspruch 11, **dadurch gekennzeichnet, daß** man aus dem Gemisch von Schmelze und Dispersion Partikel oder Granulate erzeugt.
